# EUROPEAN PATENT APPLICATION

(11) **EP 4 383 190 A1**
(43) Date of publication of application: **12.06.2024**
(21) Application number: 22290071.4
(22) Date of filing: 09.12.2022
(51) Int. Cl.: G06T 7/11, G06T 7/00, G06T 7/62

(54) **PROCESSING IMAGE DATA OF A FETUS**

(71) Applicant: Koninklijke Philips N.V., 5656 AG Eindhoven (NL)
(72) Inventor: Ciofolo, Cybèle, 5656 AG Eindhoven (NL); Olivier, Antoine, 5656 AG Eindhoven (NL); Raynaud, Caroline Denise Francoise, 5656 AG Eindhoven (NL); Germond Rouet, Laurence, 5656 AG Eindhoven (NL)
(74) Representative: Philips Intellectual Property & Standards

(57) **Abstract**

A mechanism for segmenting an amniotic fluid region from 3D image data of a uterus containing a fetus. The 3D image data is obtained (210) and processed (220) using a segmentation technique to predict the bounds of the amniotic fluid region. The amniotic fluid region is permitted to contain a portion of the limbs of the fetus.

## Description

### FIELD OF THE INVENTION

The present invention relates to the field of fetal monitoring.

### BACKGROUND OF THE INVENTION

There is an ongoing and increasing interest in performing fetal monitoring using image data. This is important for assessing the condition of the fetus and for monitoring growth of the fetus. One of the most common approaches for monitoring a fetus or fetuses is through the use of ultrasound-derived image data, or simply ultrasound image data.

During pregnancy, the volume of amniotic fluid for the fetus is a common parameter to predict or determine. This is because it can be an indicator or soft marker of pathologies such as aneuploidy. Approaches for detecting a measure of the volume of the amniotic fluid being with first predicting the bounds of an amniotic fluid region within the image data. A measure of the volume of amniotic fluid region can then be determined, either automatically or manually.

It would be advantageous to improve the speed and/or accuracy of determining the volume of amniotic fluid.

### SUMMARY OF THE INVENTION

The invention is defined by the independent claims. Dependent claims define advantageous embodiments.

According to examples in accordance with an aspect of the invention, there is provided a computer-implemented method for predicting, within 3D image data of a uterus containing a fetus, bounds of an amniotic fluid region.

The computer-implemented method comprises: obtaining 3D image data of the uterus; segmenting the 3D image data using a machine-learning method to predict the bounds of one or more regions in the 3D image data, including at least the amniotic fluid region, wherein the amniotic fluid region: contains a representation of amniotic fluid in the uterus; contains neither a representation of the placenta nor a representation of a first portion of the fetus, the first portion of the fetus excluding at least a portion of the limbs of the fetus; and is permitted to include a representation of a second portion of the fetus, the second portion including at least the portion of the limbs of the fetus.

The proposed invention provides an approach that permits a portion of the fetus, specifically a portion containing (at least) a portion of the limbs the fetus, to be included within an amniotic fluid region when performing segmentation to identify the bounds of the amniotic fluid region.

The portion of the limbs may, for each of one or more limbs of the fetus, comprise only part or the entirety of the limb. The one or more limbs may comprise all of the limbs of the fetus.

The present disclosure recognizes that the total space or volume occupied by fetal limbs is relatively small, especially relative to the overall inter-uterine volume or amniotic fluid volume. By allowing the limb(s) to form part of the amniotic fluid region, sufficiently accurate and fast identification of the bounds of an amniotic fluid region can be performed. In particular, attempting to accurately identify the bounds of moving limbs in image data is a computationally difficult and inaccurate task, and can significantly affect the quality and accuracy of identifying an amniotic fluid region that excludes limbs. This significant degradation of quality and accuracy can be avoided by allowing the amniotic fluid region to include one or limbs (e.g., ignoring the limbs entirely). The inclusion of the portion of the limbs in the amniotic fluid region does not significantly affect the accuracy of the segmentation, because the actual volume occupied by the limb(s) at a particular point in time is relatively small, compared to the volume occupied by the amniotic fluid.

The first portion of the fetus may exclude all of the limbs of the fetus. Thus, the amniotic fluid region may permit all limbs of the fetus to be included therein.

In some examples, the one or more regions includes at least a second portion region that: contains a representation of the second portion of the fetus; and does not contain a representation of the first portion of the fetus. Thus, the method may comprise segmenting the 3D image data to predict the bounds of the second portion region (e.g., the limbs). The second portion of the fetus may include all limbs of the fetus.

In some examples, the method further comprises processing the predicted bounds of the second portion region to predict a measure of the volume represented by the second portion region.

The method may further comprise controlling a user interface to provide a visual representation of the measure of the volume represented by the second portion region.

The measure of the volume of the second portion region can indicate a confidence level in the identification of the bounds of the amniotic fluid region. In particular, the larger the determined measure of the volume of the second portion region, the less certain the prediction of the bounds of the amniotic fluid (and therefore any data derived therefrom). This is because movement of the limbs will increase the predicted size of the second portion region. This movement will decrease an accuracy of the amniotic fluid region segmentation.

The step of obtaining 3D image data may comprise receiving two or more instances of initial 3D image data; and combining the two or more instances of initial 3D image data to produce the 3D image data.

The combining may comprise predicting the location of one or more predetermined landmarks in each instance of initial 3D image data; and using the predicted locations of only the one or more predetermined landmarks to fuse the instances of initial 3D image data together to thereby produce the 3D image data.

Each predetermined landmark may be independent of a movement of the portion of the limbs of the fetus relative to the remainder of the fetus.

This approach recognizes that the limbs of the fetus are more likely to move and/or have larger movements than other parts of the fetus. By preventing any landmarks (used for registering 3D image data together) from being landmarks of any the limb(s), then an accuracy in registration can be improved. This is because a movement of a landmark would cause an error or increase a chance of an error in the registration.

In some embodiments, the method further comprises processing the predicted bounds of the amniotic fluid region to predict a measure of the volume represented by the amniotic fluid region; and controlling a user interface to provide a visual representation of the predicted measure of the volume represented by the amniotic fluid region.

A measure of the amniotic fluid volume is an important parameter in assessing and/or analyzing the condition/growth of the fetus. The proposed approach provides an accurate and automated mechanism for-providing an individual or user with a suitable accurate measure of the amniotic fluid volume.

In some approaches, the one or more regions includes at least a first portion region that: contains a representation of the first portion of fetus; and does not contain a representation of the second portion of the fetus.

In an example, the one or more regions further includes a placenta region that contains a representation of a placenta for the fetus.

The method may comprise processing the predicted bounds of the first portion region to predict a measure of the volume represented by the first portion region.

The method may comprise processing the predicted bounds of the placenta region to predict a measure of the volume represented by the placenta region.

In some examples, the method may comprise defining a threshold volume measure responsive to the measure of the volume represented by the placenta region; and generating, at a user interface, a user-perceptible alert responsive to the measure of the volume represented by the first portion region falling below the threshold volume measure.

If the measure of the volume of the first portion is below a threshold defined by the placenta volume, then this can indicate that the segmentation of the first portion is inaccurate, e.g., due to fetal movement. Providing an alert can prompt the individual to repeat the capture of the image data.

The machine-learning method may be a neural network, and preferably a neural network having a U-net architecture.

The 3D image data may be 3D ultrasound image data. Other examples of 3D image data include 3D MR image data.

There is also proposed a computer program product comprising computer program code which, when executed on a processor, cause the processor to perform all of the steps of any described method.

There is also proposed a processor for predicting, within 3D image data of a uterus containing a fetus, bounds of an amniotic fluid region.

The processor is configured to obtain 3D image data of the uterus. This may be performed by an input interface of the processor and optionally a data processor, communicatively coupled to the input interface.

The processor is also configured to segment the 3D image data using a machine-learning method to predict the bounds of one or more regions in the 3D image data, including at least the amniotic fluid region. This may be performed by the data processor.

The amniotic fluid region: contains a representation of amniotic fluid in the uterus; contains neither a representation of the placenta nor a representation of a first portion of the fetus, the first portion excluding at least a portion of the limbs of the fetus; and is permitted to include a representation of a second portion of the fetus, the second portion including at least the portion of the limbs of the fetus.

The processor may be configured to receive two or more instances of initial 3D image data. This step may be performed by the input interface.

The processor is also configured to predict the location of one or more predetermined landmarks in each instance of initial 3D image data; and use the predicted locations of only the one or more predetermined landmarks to fuse the instances of initial 3D image data together to thereby produce the 3D image data, wherein each predetermined landmark is independent of a movement of the portion of the limbs of the fetus relative to the remainder of the fetus. This procedure may be performed by the data processor.

Yet another aspect of the invention provides a system comprising an imaging system for generating 3D image data of a uterus containing a fetus; and a processor as described herein.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an approach in which embodiments can be employed;
Fig. 2 is a flowchart illustrating a method according to an embodiment;
Fig. 3 is a flowchart illustrating an approach for obtaining 3D image data; and
Fig. 4 illustrates a processor according to an embodiment.

### DETAILED DESCRIPTION OF THE EMBODIMENTS

The invention will be described with reference to the drawings.

It should be understood that the detailed description and specific examples, while indicating exemplary embodiments of the invention, are intended for purposes of illustration only and are not intended to limit the scope of the invention. These and other features, aspects, and advantages of the present invention will become better understood from the following description, appended claims, and accompanying drawings. It should be understood that the drawings are merely schematic and are not drawn to scale. It should also be understood that the same reference numerals are used throughout the drawings to indicate the same or similar parts.

The invention provides a mechanism for segmenting an amniotic fluid region from 3D image data of a uterus containing a fetus. The 3D image data is obtained and processed using a segmentation technique to predict the bounds of the amniotic fluid region. The amniotic fluid region is permitted to contain a portion of the limbs of the fetus.

Embodiments are based on the realization that a movement of fetal limbs significantly impacts an accuracy in segmenting an amniotic fluid region. The present disclosure appreciates that, in practice, the total volume occupied by the fetal limbs with respect to the volume amniotic fluid is relatively small. By effectively ignoring the fetal limbs when segmenting the amniotic fluid region, a sufficiently accurate (e.g., within acceptable error margins) estimation of the bounds of the amniotic fluid region can be determined.

The identified bounds of the amniotic fluid region can be processed to determine or predict the amniotic fluid volume, e.g., using any known procedure.

Embodiments can be employed in any suitable environment in which 3D image data is produced and/or processed, such as a clinical environment when monitoring a fetus or performing any other obstetrics-based procedure.

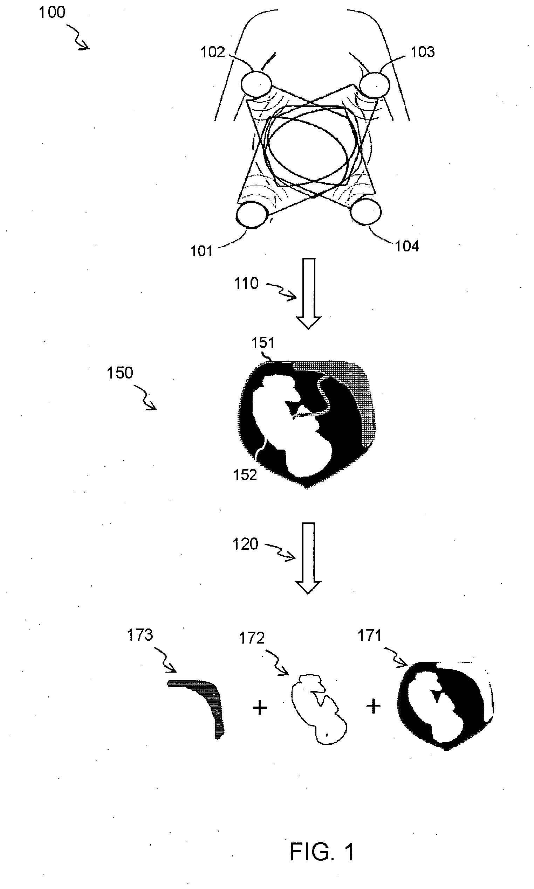

Fig. 1 conceptually illustrates an approach in which embodiments according to the present disclosure can be employed. This is provided for improved contextual understanding of the proposed approach.

Initially, 3D image data 150 of a uterus 151 containing a fetus 152 is obtained; a placenta is shown in the upper-right corner. The 3D image data may be captured or otherwise acquired by an imaging system. The 3D image data may, for instance, be a 3D image of the uterus containing the fetus.

Suitable examples of imaging systems for obtaining 3D image data are well known to the skilled person. Preferably, such imaging systems do not make use of ionizing radiation, such as X-rays. One suitable example is an ultrasound imaging system, but another option is a MR imaging system.

The illustrated procedure for obtaining 3D image data includes acquiring (by an imaging system) multiple 3D acquisitions of the uterus from different points of view 101, 102, 103, 104. Thus, a plurality of instances of initial 3D image data (e.g., initial 3D images) of the uterus may be obtained, e.g., where each instance represents a different imaging view or angle. The instances of initial 3D image data can then be fused or blended together, e.g., in a fusion process 110, to produce the 3D image data.

Various approaches for performing fusion of different instances of initial 3D image data could be used.

In some examples, one or more landmarks in the instances of initial 3D image data are used in the fusion process. Those (one or more) landmarks can be anatomical landmarks of the fetus (e.g., head structures, abdominal structures, bones), uterus and/or placenta (contained in the uterus). Identification of these landmarks can be used to ensure or maintain known anatomical characteristics. For instance, in the 3D image data produced by the fusion data, structures such as the uterine wall or the placenta contour should be continuous.

EP 3,711,677 A1 discloses an approach for fusing or blending 3D ultrasound images together, which makes use of one or more identified landmarks or identified anatomical features.

In some approaches, if the initial positions of an ultrasound probe (used to acquire the image data) are known, e.g., when the instances of initial 3D (ultrasound) image data have been acquired from pre-defined points of view or if a probe tracker was available, then these positions can be used as an initialization of the fusion step.

As an alternative to fusing instances of initial 3D image data to produce the 3D image data, 3D image data may be captured in a single image capturing process (e.g., rather than capturing multiple instances).

Regardless of how the 3D image data is obtained or produced, the 3D image data can then be processed using a segmentation process 120 in order to identify/predict the bounds of an amniotic fluid region 171 (containing a representation of (only) the amniotic fluid) within the 3D image data. The segmentation process may additionally identify the bounds of a fetal region 172 (containing a representation of (only) the fetus) and/or a placenta region 173 (containing a representation of (only) the placenta) within the 3D image data.

The segmentation process may be performed by using a machine-learning method. Machine-learning methods that are capable of performing segmentation of 3D image data are well established in the art.

One example of a suitable machine-learning method is a 3D version of a segmentation neural network, such as that proposed by Ronneberger, O., Fischer, P., & Brox, T. (2015). U-Net: Convolutional networks for biomedical image segmentation. MICCAI'15, (pp. 234-241).

Another example is disclosed by Prieto, Juan C., et al. "An automated framework for image classification and segmentation of fetal ultrasound images for gestational age estimation." Medical Imaging 2021: Image Processing. Vol. 11596. SPIE, 2021.

Such machine-learning methods may provide an output channel for each feature segmented (e.g., a channel for the amniotic fluid and optional channel(s) for the placenta and/or fetus).

Other suitable segmentation processes are known in the art, e.g., that make use of deformable models. Other example segmentation processes are described by EP 3,852,062 A1 or EP 3,493,154 A1.

Once the bounds of an amniotic fluid region within 3D image data have been predicted, it is possible to process the predicted bounds of the amniotic fluid region to determine or predict a volume of amniotic fluid within the uterus. As the inter-voxel distance (i.e., the physical distance between adjacent voxels) and/or voxel size (i.e., the total size of the physical, space represented by a single voxel) is well established in the art of imaging systems, simple geometrical calculations can be used to process the identified bounds of the amniotic fluid region to determine the size of the amniotic fluid region. The determined size may, for instance, be calculated and/or expressed using any suitable unit of measurement, e.g., mm³, cm³, ml, cl, l and so on.

The present disclosure proposes an approach for improving the robustness of segmenting an amniotic fluid region from 3D image data of a uterus containing a fetus. The proposed technique differs from existing approaches in that the amniotic fluid region is permitted to contain a portion of the fetus that contains at least a portion of the limbs of the fetus. Thus, one or more fetal limbs are effectively ignored during the segmentation of the amniotic fluid region with respect to the 3D image data. In a particular example, if a neural network or other machine-learning method is used, the machine-learning method is trained to effectively ignore the fetal limbs localization, for example by including images with various fetal limb positions in the training dataset.

It has been recognized that the size of fetal limbs is small, so as to be negligible compared to the volume occupied by amniotic fluid. In particular, the fetal limbs do not represent a large proportion of the total intra-uterine volume. Thus, a sufficiently accurate identification of the bounds of the amniotic fluid region (for medical/clinical purposes) can be predicted whilst ignoring the fetal limb(s).

Moreover, it has been recognized that one significant difficulty in accurately segmenting 3D image data (of a uterus) results from limb movements. Due to the nature of how 3D image data is obtained/captured, limb movements can result in significant shadows or artefacts (e.g., motion blur) in the 3D image data. For instance, if the movements are large, then the region of the 3D image that appears to be occupied by the limbs in the 3D image may be significantly larger than the true occupation size by the limbs.

By ignoring the fetal limbs in the segmentation of the amniotic fluid region, i.e., by allowing the limb(s) to be contained in the amniotic fluid region, the effect of this movement on the segmentation of the amniotic fluid region is entirely avoided, whilst still defining sufficiently accurate bounds for the amniotic fluid region (e.g., for calculating a volume of the same within an acceptable margin of error).

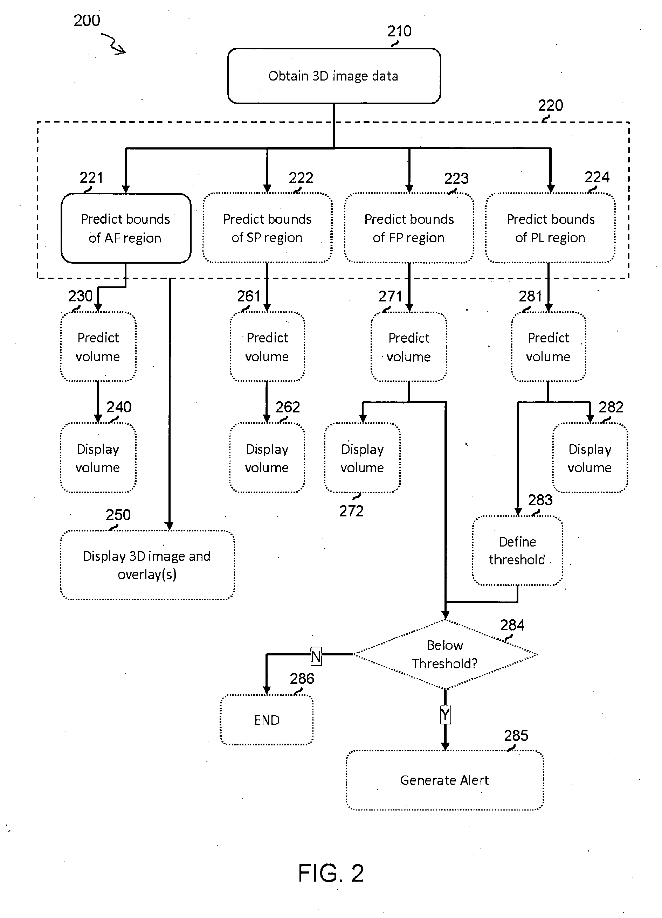

Fig. 2 illustrates a computer-implemented method 200 according to an embodiment of the invention. The method 200 is configured for predicting, within 3D image data of a uterus containing a fetus, the bounds of an amniotic fluid region. The method 200 may, for instance, be performed by a processor.

The computer-implemented method comprises a step 210 of obtaining 3D image data, e.g., a 3D image, of the uterus.

Preferably, the 3D image data is 3D ultrasound image data, as this is widely used in the field of fetal monitoring and/or screening such that the proposed approach is particularly advantageous when adapted for use with ultrasound image data. One alternative to 3D ultrasound image data includes 3D MR imaging data.

Step 210 may comprise, for instance using an imaging system to image/capture the 3D image data of the uterus. Alternatively, obtaining the 3D image data may comprise retrieving (e.g., via an input interface) the 3D image data from a memory and/or storage unit, e.g., previously captured and stored by an imaging system. Other examples for performing step 210 are provided later in this disclosure.

The computer-implemented method 200 further comprises a step 220 of segmenting the 3D image data using a machine-learning method to predict the bounds of one or more regions in the 3D image data, including at least the amniotic fluid region. Approaches for performing segmentation of 3D image data have been previously described.

Accordingly, step 220 conceptually includes a sub-step 221 of generating or producing the predicted bounds of the amniotic fluid region (AF region).

The amniotic fluid region contains a representation of amniotic fluid in the uterus; contains neither a representation of the placenta nor a representation of a first portion of the fetus, the first portion excluding a portion of the limbs of the fetus; and is permitted to include a representation of a second portion of the fetus, the second portion including the portion of the limbs of the fetus.

The portion of the limbs may comprise at least a portion or part of each of one or more limbs of the fetus, e.g., the entirety of each of a one or more limbs. The one or more limbs may comprise only the arms of the fetus, only the legs of the fetus and/or all of the limbs of the fetus. In this way, the first portion of the fetus may exclude all of the limbs of the fetus. Thus, the amniotic fluid region may be permitted to contain a representation of any and/or all limbs of the fetus represented in the 3D image data.

Thus, the predicted bounds of the amniotic fluid region aim to capture the representation of the amniotic fluid, without capturing the representation of the placenta and/or part of the fetus. The representation of the fetal limb(s) is ignored for the purposes of predicting the bounds, i.e., the predicted bounds for the amniotic fluid region do not target excluding capture of at least a portion of the fetal limb(s), e.g., all of the fetal limbs.

In some examples, the amniotic fluid region is configured to include at least a portion of the limbs of the fetus, e.g., a portion/part or the entirety of all limbs of the fetus. This can be performed through appropriate configuration and/or training of the machine-learning method (e.g., of the training dataset used to train the machine-learning method).

It has previously been described how segmentation of 3D image data (to predict the bounds of one or more regions represented by the 3D image data) is performed using a machine-learning method.

A machine-learning algorithm is any self-training algorithm that processes input data in order to produce or predict output data. Here, the input data comprises 3D image data and the output data comprises the predicted bounds (within the 3D image data) of one or more regions within the 3D image data (including at least the amniotic fluid region).

Suitable machine-learning algorithms for being employed in the present invention will be apparent to the skilled person, and a number of suitable examples (e.g., neural networks) have been previously identified. A particularly advantageous machine-learning method for use in the present disclosure is a neural network having a U-net architecture, as this has been shown to be particularly effective at performing an image segmentation task.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±X%) to the training output data entries. The value of X may be predetermined, e.g., 1 or 0.5, but may depend upon which metric is used, which can vary depending upon the embodiment. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example instances of 3D image data. The training output data entries correspond to bounds of the amniotic fluid region within the 3D image data of the corresponding training input data entry.

It is possible to train a machine-learning algorithm to predict the bounds of an amniotic fluid region that permits the inclusion of at least a portion of the fetal limbs through appropriate selection and/or generation of the training dataset. In particular, each training output data entry may be configured such that the bounds of the amniotic fluid region (of that training output data entry) include or incorporate the representation of at least a portion of the fetal limbs in the 3D image data (of the corresponding training input data entry). The identification of the bounds of the amniotic fluid region for the training output data entry may be performed by an appropriately skilled/experienced clinician or analyst.

The skilled person would be readily capable of preparing or providing a training dataset that could be used to train a machine-learning algorithm to identify the bounds of one or more other regions (e.g., a placenta region and/or a fetal region) within the 3D image data, using similar principles.

The method 200 may further comprise a step 230 of processing the predicted bounds of the amniotic fluid region to predict a measure of the volume represented by the amniotic fluid region. This volume can be labelled an amniotic fluid volume, such that step 230 comprises processing the predicted bounds of the amniotic fluid region with respect to the 3D image to determine or predict an amniotic fluid volume.

Approaches for performing step 230 will be readily apparent to the skilled person, e.g., using geometric-based volume determination steps and/or pixel/voxel counting steps in order to calculate the (measure of) volume of a region within a 3D image.

The method 200 may further comprise a step 240 of controlling a user interface to provide a visual representation of the predicted measure of the volume represented by the amniotic fluid region.

Approaches for controlling a user interface to provide visual representations of information are well-established in the art, and are not disclosed for the sake of conciseness.

It is recognized that the measure of a volume of the amniotic fluid is an important parameter or property in assessing and/or analyzing fetal growth and/or pregnancy progress. In particular, the measure of the volume of amniotic fluid facilitates determination or detection of polyhydramnios or oligohydramnios, both of which are associated with fetal/congenital abnormalities as well as other problems during pregnancy.

Providing a user with a sufficiently accurate measure of the volume of amniotic fluid therefore aids a clinician in making a clinical decision about the fetus and/or pregnant individual.

Although advantageous, it is not essential that step 240 be performed when step 230 is carried out. Rather, the predicted measure of the volume of the amniotic fluid may instead be stored in a memory/storage system and/or provided to a further processor for processing (e.g., for automated assessment of the condition of the fetus and/or pregnant individual).

Of course, the method may further comprise a step 250 of controlling a/the user interface (e.g., if performed, the same user interface as controlled in step 240) to provide a visual representation of the 3D image with an overlay identifying the predicted bounds of at least one of the one or more regions, e.g., the predicted bounds of the amniotic fluid region, with respect to the 3D image.

This provides a clinician with a useful indicator of the amniotic fluid region with respect to the 3D image, to improve an ease of identifying features or elements of the amniotic fluid region.

As previously explained, the proposed approach recognizes that movement of the limb(s) can cause artefacts in the 3D image data. By ignoring at least a portion of the limb(s) in the segmentation of the amniotic fluid region, the effect of this movement is mitigated.

The portion of the limbs may contain a portion of the arms and/or legs of the fetus. In preferred examples, the portion of the limbs contains at least a portion of the legs of the fetus (as these tend to have a larger magnitude of movement than the arms of the fetus). The portion of the limbs may contain the entirety of one or more limbs (e.g., the entirety of the legs and/or arms) or only a part of the limbs (e.g., the hands and/or feet of the fetus or the outermost portion of the limbs of the fetus).

Various optional modifications to the above-described steps and/or additional optional steps, which confer additional advantages, for the method 200 are hereafter described.

In some embodiments, the one or more regions (whose bounds are predicted in step 220) includes at least a second portion region (SP region). The second portion region contains a representation of the second portion of the fetus (which includes at least the portion of the limbs of the fetus) and does not contain a representation of the first portion of the fetus.

Thus, the second portion region includes a representation of at least the one or more limbs of the fetus that are permitted to be included in the amniotic fluid region.

In this way, step 220 may conceptually include a sub-step 222 of generating or producing the predicted bounds of the second portion region.

The method 200 may correspondingly comprise a step 261 of processing the predicted bounds of the second portion region to predict a measure of the volume represented by the second portion region.

The method 200 may further comprise a step 262 of controlling a/the user interface (e.g., if performed, the same user interface as controlled in steps 240 and/or 250) to provide a visual representation of the measure of the volume represented by the second portion region. Alternatively and/or additionally, the method may output the measure of the volume of the second portion region to a memory and/or further processor.

It has been advantageously recognized that the predicted size/volume of the second portion of the fetus (e.g., the size of the limbs of the fetus) represents an accuracy and/or error of the prediction of the bounds of the amniotic fluid region (and consequently, if determined, the determined volume of the amniotic fluid region).

By providing the predicted size/volume of the second portion of the fetus at a user interface, a clinician is able to understand a confidence and/or accuracy of the measurement of the volume of the amniotic fluid region. This provides valuable information for a clinician in making a clinical decision (e.g., as to whether a further/repeat imaging of the uterus is required or whether the volume still fall within acceptable bounds despite its inaccuracy).

It will be appreciated that the size of the second portion region is influenced or responsive to a movement of the limb(s). In particular, the larger the movement, the larger the apparent volume occupied by the limb(s), due to motion blurring as a consequence of how the 3D image data is constructed/captured. More particularly, not all data elements in 3D image data are captured simultaneously (especially if the 3D image data is created by fusing instances of initial 3D image data), which results in apparent motion blur. The movement of the limb(s) will also affect the accuracy of the segmentation of the amniotic fluid region, such that the predicted size of the second portion region provides accuracy information from another perspective.

In some embodiments, the one or more regions (whose bounds are predicted in step 220) includes at least a first portion region (FP region) that contains a representation of the first portion of fetus and does not contain a representation of the second portion of the fetus.

Thus, step 220 may conceptually include a sub-step 223 of generating or producing the predicted bounds of the first portion region.

Correspondingly, the method 200 may comprise a step 271 of processing the predicted bounds of the first portion region to predict a measure of the volume represented by the first portion region ("first region volume").

Similarly, the method 200 may comprise a step 272 of controlling a/the user interface (e.g., if performed, the same user interface as controlled in steps 240, 250 and/or 262) to provide a visual representation of the measure of the volume represented by the first portion region. Alternatively and/or additionally, the method may output the measure of the volume of the first portion region to a memory and/or further processor.

In some embodiments, the one or more regions (whose bounds are predicted in step 220) includes at least a placenta region (PL region) that contains a representation of a placenta for the fetus.

Thus, step 220 may conceptually include a sub-step 224 of generating or producing the predicted bounds of the placenta region.

Correspondingly, the method 200 may comprise a step 281 of processing the predicted bounds of the placenta region to predict a measure of the volume represented by the placenta region ("placenta volume").

Similarly, the method 200 may comprise a step 282 of controlling a/the user interface (e.g., if performed, the same user interface as controlled in steps 240, 250, 262 and/or 272) to provide a visual representation of the measure of the volume represented by the second portion region. Alternatively and/or additionally, the method may output the measure of the volume of the placenta region to a memory and/or a further processor.

If both steps 281 and 282 are performed, then the method 200 may also perform steps 283-285.

Step 283 comprises setting a threshold volume measure responsive to the measure of the volume represented by the placenta region. The threshold volume measure should be a (minimum) expected measure for a volume of the first portion region (e.g., a minimum expected volume of the fetus) for the volume of the placenta region.

Existing medical guidance sets out an expected relationship or mapping between a fetal volume and a placenta volume. Similarly, medical datasets would provide examples of expected first portion volumes and placenta volumes. In particular, there is an expected relationship between fetal size and the size of the placenta. This information can be used to set the threshold volume measure. For instance, a placenta volume may be associated (by medical guideline or a medical database) with known/expected potential fetal volumes. The Xth percentile potential fetal volume associated with the predicted placenta volume may be set as the threshold. X may be a predetermined value, e.g., 10, 5 or 1.

In some examples, the threshold volume measure can be determined when training the segmentation algorithm, e.g., using the training dataset.

Step 284 comprises determining whether or not the measure of the volume represented by the first portion region (i.e., the first portion volume) falls below the threshold defined in step 283.

Step 285 comprises generating, at a user interface, a user-perceptible alert responsive to the measure of the volume represented by the first portion region falling below the threshold volume measure. The user-perceptible alert is preferably a visual alert (e.g., a pop-up message and/or a flashing light or indicator), but may otherwise and/or additionally be an audible alert and/or a haptic alert.

If the first portion volume is at or above the first portion region, no further action needs to be taken, e.g., the method may end in step 286.

It has been recognized that if the determined first portion volume is too small compared to the determined placenta volume, this may mean that correct segmentation of the fetus has failed. This may, for instance, be due to fetal movement involving the head and/or abdomen of the fetus. Thus, an alert (e.g., a message or a warning) can be displayed to the clinician, e.g., to prompt the clinician to repeat the acquisition of the 3D image data.

Put another way, if the first portion volume is below a threshold defined by the placenta volume, then this can indicate that the segmentation of at least the first portion is inaccurate, e.g., due to fetal movement. Providing an alert can prompt the individual to repeat the capture of the image data.

Step 250, if performed, may comprise controlling the user interface (e.g., if performed, the same user interface as controlled in step 240) to provide a visual representation of the 3D image with an overlay identifying the predicted bounds of all predicted regions with respect to the 3D image. Thus step 250 may provide (at the user interface) an overlay on a visual representation of the 3D image data for each region whose bounds are predicted in step 220.

This would provide a clinician with extremely valuable information for understanding and/or assessing the 3D image data, e.g., to assess the spatial relationship between various features of the fetus/uterus and/or performing a size comparison. Such information is important in assessing the condition of the fetus and/or pregnant individual.

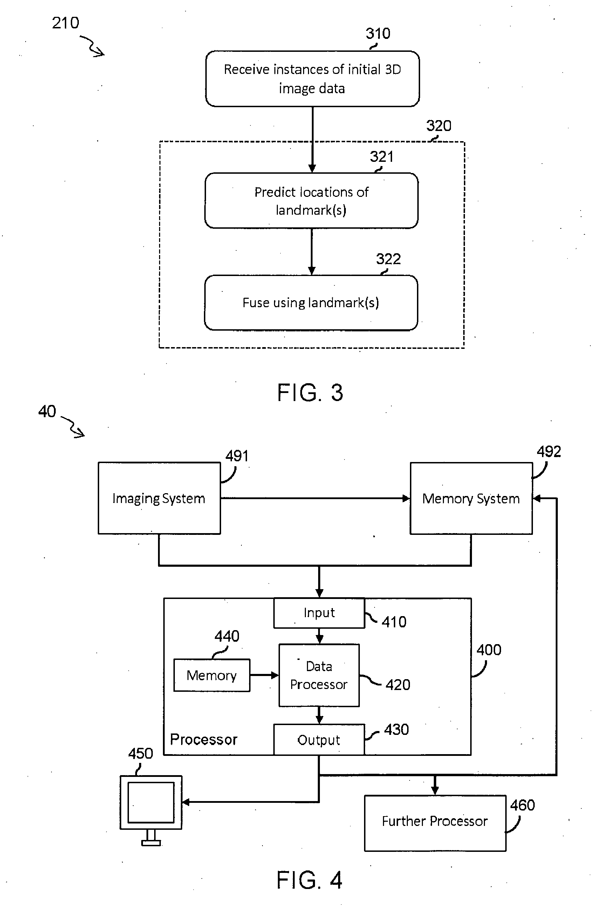

Fig. 3 is a flowchart illustrating an approach for performing step 210 of obtaining of 3D image data according to an embodiment.

The step 210 may comprise a step 310 of receiving two or more instances of initial 3D image data. Each instance of initial 3D image data may represent a different portion and/or viewpoint of a uterus containing a fetus.

The step 210 also comprises a step 320 of combining the two or more instances of initial 3D image data to produce the 3D image data. Thus, the two or more instances of initial 3D image data are fused or blended to produce the 3D image data for later processing.

Approaches for fusing or 3D image data to produce fused/combined 3D image data have been previously described.

In a particularly advantageous embodiment, step 320 comprises a step 321 predicting the location of one or more predetermined landmarks in each instance of initial 3D image data. Thus, each instance of initial 3D image data may be processed in step 321 to identify the location of one or more landmarks, such as anatomical landmarks.

Approaches for identifying landmarks in 3D image data are well established in the art. One approach, which makes use of a machine-learning approach, is disclosed/suggested by EP 3,655,917 B1.

The locations of the identified landmark(s) can then be used to fuse the instances of initial 3D image data together, in step 322, to produce the 3D image data. Approaches that fuse image data together using landmarks are established in the art. In particular, the landmarks can be used to register the instances of image data with respect to one another, and to then combine non-overlapping parts of each instance of image data (and selecting from one of the overlapping parts and/or interpolating image data of overlapping parts) to produce the 3D image data. Using interpolation of overlapping parts, rather than selecting a single one of the overlapping parts can increase the image resolution by taking advantage of the different parts.

An approach for performing steps 321 and 322 is disclosed in EP 3,934,539 A1. Other approaches will be apparent to the skilled person.

In particularly advantageous embodiments, each predetermined landmark (whose location is predicted in step 321) is independent of a movement of the portion of the limbs of the fetus relative to the remainder of the fetus.

Thus, each predetermined landmark may, for instance, not include any landmark present on any limb of the fetus - such as a location of a hand or foot. Rather, the landmarks may be located on any non-limb portion of the fetus (such as the head, the abdomen, the heart and so on) or on any non-fetus portion of the 3D image data.

It has been recognized that there may be significant movement of the limbs between the capture of different instances of 3D image data. Thus, landmarks that are positioned on the limbs are likely to move between the captures of the instances of 3D image data. This would significantly affect an accuracy of registering different instances of 3D image data together based on such landmarks. By preventing the landmarks from being located on the limb of the fetus, this problem can be avoided and the accuracy of fusing/blending the instances of 3D image data together can be significantly improved.

It will be understood that disclosed methods are preferably computer-implemented methods. Thus, different portions, lines or blocks of code of a computer program according to an embodiment may be executed by a processor to perform any herein described method.

As such, there is also proposed the concept of a computer program comprising code for implementing any described method when said program is run on a processor, such as a computer. Put another way, there is proposed a computer program product comprising computer program code which, when executed on a computing device having a processor, cause the processor to perform any herein described method.

The computer program product may include a storage medium (e.g. non-transitory) configured to store one or more computer programs or code that, when executed, perform any herein described method. Alternatively, the computer program product is formed by software that can be downloaded from a server (e.g. using the internet).

There is also proposed a processor for predicting, within 3D image data of a uterus containing a fetus, bounds of an amniotic fluid region.

Fig. 4 illustrates a system 40 comprising a processor 400 according to an embodiment. The system 40 also represents an embodiment of the invention.

The processor 400 may comprise an input interface 410, a data processor 420 and an output interface 430. The input interface and the data processor are communicatively coupled together. Similarly, the output interface and the data processor are communicatively coupled together.

The processor 400 is configured to obtain 3D image data of the uterus. This may be performed by an input interface 410 of the processor 400 and, optionally, a data processor 420 of the processor 400.

In one embodiment, the input interface 410 directly receives the 3D image data from an imaging system 491 and/or a memory system 492 of the system 40. In an embodiment, the imaging system 491 is an ultrasound imaging system. In other embodiments, other imaging techniques like MRI, CT or X-ray are used in the imaging system 491. In an embodiment, the memory system 492 includes a solid-state memory. In other embodiments, the memory system includes one or more hard disk drives, one or more optical disk drives (e.g. CD-RW or DVD-RW), and/or a magnetic tape-based memory system, and/or a transitory memory like RAM.

In another embodiment, the input interface 410 receives a plurality of instances of initial 3D image data from the imaging system 491 and/or the memory system 492 of the system 40. The data processor 420 may then process the plurality of instances of initial 3D image data to produce the 3D image data.

For instance, the data processor 420 may predict the location of one or more predetermined landmarks in each instance of initial 3D image data; and using the predicted locations of only the one or more predetermined landmarks to fuse the instances of initial 3D image data together to thereby produce the 3D image data. Each predetermined landmark may be independent of a movement of the one or more limbs of the fetus relative to the remainder of the fetus.

In some examples, the processor 400 controls (using the data processor 420 and via the input interface 410) the operation of the imaging system 491 in order to control the acquisition of the 3D image data and/or the instances of initial 3D image data. Approaches for controlling an imaging system are well established in the art.

However, this is not essential, and the processor may instead passively receive or request to receive the 3D image data and/or the instance of initial 3D image data.

The processor 400 is also configured to segment the 3D image data using a machine-learning method to predict the bounds of one or more regions in the 3D image data, including at least the amniotic fluid region. Approaches for performing this process have been previously described.

This segmentation procedure may be performed by the data processor 420, e.g., by executing software stored in a memory 440.

The data processor 420 can be virtually any custom made or commercially available processor, a graphics processing unit (GPU), a central processing unit (CPU), a digital signal processor (DSP), or an auxiliary processor among several processors associated with the processor 400, and the processor 420 may be a semiconductor-based microprocessor (in the form of a microchip) or a microprocessor. Alternatively, the data processor may be or include dedicated hardware, like an application-specific integrate circuit (ASIC) or a field-programmable gate array (FPGA).

The memory 440 can include any one or combination of volatile memory elements (e.g., random access memory (RAM), such as dynamic random access memory (DRAM), static random access memory (SRAM), etc.) and nonvolatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 440 may incorporate electronic, magnetic, optical, and/or other types of storage media. Note that the memory 440 can have a distributed architecture, where various components are situated remote from one another, but can be accessed by the processor 420.

The software in the memory 440 may include one or more separate programs, each of which comprises an ordered listing of executable instructions for implementing logical functions. The software in the memory 440 may include a suitable operating system (O/S), compiler, source code, and one or more applications (e.g., configured, when run, to perform any herein disclosed method) in accordance with exemplary embodiments. The software may also be located remotely on a cloud.

The application comprises numerous functional components for implementing the features and operations of the exemplary embodiments. The application of the computer may represent various applications, computational units, logic, functional units, processes, operations, virtual entities, and/or modules in accordance with exemplary embodiments, but the application is not meant to be a limitation.

The application may be a source program, executable program (object code), script, or any other entity comprising a set of instructions to be performed. When a source program, then the program is usually translated via a compiler (such as a compiler), assembler, interpreter, or the like, which may or may not be included within the memory 440, so as to operate properly in connection with the O/S. Furthermore, the application can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The processor 400 may be appropriately adapted to perform any herein described procedure.

In particular, the processor 400 may be configured to process the predicted bounds of the amniotic fluid region to predict a measure of the volume represented by the amniotic fluid region. This can be performed by the data processor 420.

Similarly, the processor 400 may be configured to control a user interface 450 to provide a visual representation of the predicted measure of the volume represented by the amniotic fluid region.

This can be performed by the data processor 420 via the output interface 430.

Of course, any data produced or generated by the data processor 420 may be stored, e.g., via the output interface 430, in the memory system 492 and/or passed to a further processor 460.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

Measures recited in mutually different dependent claims may advantageously be combined.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A computer-implemented method (200) for predicting, within 3D image data (150) of a uterus (151) containing a fetus (152), bounds of an amniotic fluid region, the computer-implemented method comprising:
obtaining (210) 3D image data of the uterus;
segmenting (220, 120) the 3D image data using a machine-learning method to predict the bounds of one or more regions in the 3D image data, including at least the amniotic fluid region (171),
wherein the amniotic fluid region:
contains a representation of amniotic fluid in the uterus;
contains neither a representation of the placenta nor a representation of a first portion of the fetus, the first portion of the fetus excluding at least a portion of the limbs of the fetus; and
is permitted to include a representation of a second portion of the fetus, the second portion including at least the portion of the limbs of the fetus.

2. The computer-implemented method of claim 1, wherein the first portion of the fetus excludes all of the limbs of the fetus.

3. The computer-implemented method of claim 1 or 2, wherein the one or more regions includes at least a second portion region that:
contains a representation of the second portion of the fetus; and
does not contain a representation of the first portion of the fetus.

4. The computer-implemented method of claim 3, further comprising:
processing (261) the predicted bounds of the second portion region to predict a measure of the volume represented by the second portion region; and
controlling (262) a user interface to provide a visual representation of the measure of the volume represented by the second portion region.

5. The computer-implemented method of any of claims 1 to 4, wherein the step (210) of obtaining 3D image data comprises:
receiving (310) two or more instances of initial 3D image data; and
combining (320 the two or more instances of initial 3D image data to produce the 3D image data.

6. The computer-implemented method of claim 5, wherein the combining (320) comprises:
predicting (321) the location of one or more predetermined landmarks in each instance of initial 3D image data; and
using the predicted locations of only the one or more predetermined landmarks to fuse (322) the instances of initial 3D image data together to thereby produce the 3D image data,
wherein each predetermined landmark is independent of a movement of the portion of the limbs of the fetus relative to the remainder of the fetus.

7. The computer-implemented method of any of claims 1 to 5, further comprising:
processing (230) the predicted bounds of the amniotic fluid region to predict a measure of the volume represented by the amniotic fluid region; and
controlling (240) a user interface to provide a visual representation of the predicted measure of the volume represented by the amniotic fluid region.

8. The computer-implemented method of any of claims 1 to 7, wherein the one or more regions includes at least a first portion region that:
contains a representation of the first portion of fetus; and
does not contain a representation of the second portion of the fetus.

9. The computer-implemented method of any of claims 1 to 8, wherein the one or more regions further includes a placenta region that contains a representation of a placenta for the fetus.

10. The computer-implemented method of claim 9, when dependent upon claim 8, further comprising:
processing (271) the predicted bounds of the first portion region to predict a measure of the volume represented by the first portion region;
processing (272) the predicted bounds of the placenta region to predict a measure of the volume represented by the placenta region.

11. The computer-implemented method of claim 10, further comprising:
defining (283) a threshold volume measure responsive to the measure of the volume represented by the placenta region; and
generating (285), at a user interface, a user-perceptible alert responsive to the measure of the volume represented by the first portion region falling below the threshold volume measure.

12. The computer-implemented method of any of claims 1 to 11, wherein the 3D image data is 3D ultrasound image data.

13. A computer program product comprising computer program code which, when executed on a processor, cause the processor to perform all of the steps of the method according to any of claims 1 to 12.

14. A processor (400) for predicting, within 3D image data of a uterus containing a fetus, bounds of an amniotic fluid region, the processor being configured to carry out the method of any of claims 1 to 12.

15. A system (40) comprising
an imaging system (491) for generating 3D image data (150) of a uterus (151) containing a fetus (152); and
a processor (400) as claimed in claim 14.
